# EUROPÄISCHE PATENTSCHRIFT

(11) **EP 2 839 847 B1**
(45) Veröffentlichungstag und Bekanntmachung des Hinweises auf die Patenterteilung: **16.09.2015**
(21) Anmeldenummer: 14000206.4
(22) Anmeldetag: 21.01.2014
(51) Int. Cl.: A61L 2/26, A61B 19/02

(54) **Filterhalterung für Sterilcontainer**
Filter holder for a sterile container
Support de filtre pour conteneurs stériles

(30) Priorität: 23.08.2013 DE 202013007581 U
(43) Veröffentlichungstag der Anmeldung: 25.02.2015
(73) Patentinhaber: Innovations Medical GmbH, 78532 Tuttlingen (DE)
(72) Erfinder: Kreidler, Winfried, 78532 Tuttlingen (DE); Kreidler, Jochen, 78532 Tuttlingen (DE)
(74) Vertreter: Binner, Bernhard

(56) Entgegenhaltungen:
- EP-A1- 1 563 854
- EP-A1- 2 478 921
- WO-A1-03/041748
- WO-A1-03/041749

## Beschreibung

Die Erfindung betrifft eine Filterhalterung mit einem Sterilcontainer, welcher in einem Wandbereich oder in mehreren Wandbereichen mit Belüftungsöffnungen versehen ist, durch welche im geschlossenen Zustand ein Luftaustausch zwischen dem Innenraum des Sterilcontainers und der Umgebung stattfindet, wobei die Belüftungsöffnungen mittels eines Filterblattes zur Bildung einer Sterilbarriere abgedeckt sind, welche eine Sterilisation der durch die Belüftungsöffnungen in den Sterilraum gelangenden Luft bewirkt, wobei das Filterblatt durch eine luftdurchlässige Andruckscheibe auf dem Wandbereich gehalten ist, wobei im Wandbereich der Belüftungsöffnungen ein über den Wandbereich vorstehender, feststehend mit dem Wandbereich in Verbindung stehender Lagerzapfen vorgesehen ist.

Sterilcontainer oder auch Sterilisierbehälter genannt, bestehen in der Regel aus einem kastenartigen, unteren Behälterteil und einem dicht darauf aufsetzbaren Behälterdeckel. Solche Sterilcontainer werden bekannterweise zur Sterilisation von medizinischen Geräten eingesetzt. In der Regel findet dabei während des Sterilisiervorganges eine Erwärmung des kompletten Sterilcontainers statt, so dass ein diverser Überdruck innerhalb des Containers entsteht. Um diesen Überdruck abbauen zu können, sind solche Sterilcontainer in einem Wandbereich oder in mehreren Wandbereichen mit Belüftungsöffnungen versehen, durch welche der Luftaustausch mit der Umgebung stattfindet.

Um insbesondere in der Abkühlphase, in welcher Umgebungsluft in den Sterilcontainer zum Druckausgleich eindringt, eine Sterilisierung dieser eindringenden Umgebungsluft zu bewirken, sind solche Sterilcontainer in der Regel mit einer sogenannten Sterilbarriere ausgestattet. Bei einigen Ausführungsvarianten dient als Sterilbarriere ein luftdurchlässiges Filterblatt, welches die Belüftungsöffnungen vollständig abdeckt. Um ein solches Filterblatt im Wandbereich der Belüftungsöffnungen festsitzend und dicht zu fixieren, sind diverse Konstruktionen aus dem Stand der Technik bekannt, bei welchen zur Halterung dieses Filterblattes eine luftdurchlässige Andruckscheibe eingesetzt wird.

Hierzu sei beispielsweise auf die DE 20 2010 009 925 U1 verwiesen. Bei dieser Konstruktion wird ein Rahmenelement verwendet, welches ringförmig ausgebildet ist und feststehend im Umgebungsbereich der Belüftungsöffnungen innenseitig im entsprechenden Wandbereich angeordnet ist. Bei dieser Konstruktion weist die Andruckscheibe diverse Durchbrüche auf, so dass die Andruckscheibe selbst ebenfalls luftdurchlässig ist. Zur Halterung der Andruckscheibe bzw. zum Anpressen gegen das auf die Innenwand im Bereich der Belüftungsöffnungen aufgelegte Filterblatt ist bei dieser Konstruktion eine Federscheibe vorgesehen, welche mit Renkverbindungselementen versehen ist. Mittels dieser Renkverbindungselemente ist die Federscheibe mit entsprechend zugeordneten Renkverschlusselementen des Rahmenelements in und außer Eingriff bringbar. Des Weiteren ist die Federscheibe gegenüber der Andruckscheibe um eine gemeinsame zentrale Drehachse drehbar, wobei die Andruckscheibe im Rahmenelement während der drehenden Verstellung der Federscheibe unverdrehbar gehalten wird.

Bei dieser Konstruktion wird somit eine Vielzahl von Bauteilen eingesetzt, um ein Filterblatt feststehend gegen die Innenwand des Wandbereiches mit den Belüftungsöffnungen pressen zu können.

Des Weiteren ist aus der DE 20 2011 001 772 U1 eine weitere Konstruktion bekannt, bei welcher die Andruckscheibe mittels spezieller Rastverbindungen über eine Kugelrastverbindung oberseitig gegen das Filterblatt drückt. Bei dieser Konstruktion ist die Andruckscheibe als runde Scheibe ausgebildet und weist außenseitig eine umlaufende Rastnut auf, mit welcher die Andruckscheibe rastend mit Rastelementen in Verbindung bringbar ist.

Diese Rastelemente sind hierzu im Umfangsbereich der Belüftungsöffnungen und somit der montierten Andruckscheibe in gleichmäßiger Verteilung angeordnet. Dabei ist jedes Rastelement als separates Bauteil ausgebildet und in rechtwinklig zum Wandbereich verlaufenden Aufnahmebohrungen von axial über den Wandbereich vorstehenden Ringsegmenten feststehend angeordnet.

Die Rastelemente werden aus einem etwa zylindrischen Gehäuseblock gebildet, welcher mit einer quer verlaufenden Radialbohrung versehen ist. In dieser Radialbohrung ist eine Rastkugel federbelastet verstellbar aufgenommen, welche im montierten Zustand radial nach innen in die Rastnut der Andruckscheibe im montierten Zustand eingreift. Dabei wird der Gehäuseblock des Rastelementes beispielsweise mittels eines Spannstiftes in der jeweiligen Aufnahmebohrung feststehend fixiert.

Vorteilhaft bei diesen beiden Gegenständen der DE 20 2010 009 925 U1 und DE 20 2011 001 722 U1 ist, dass die Andruckscheibe auf das Filterblatt pressbar ist, ohne dass die Andruckscheibe zur Fixierung relativ zum Filterblatt gedreht werden muss, wie dies bei früheren Konstruktionen aus dem Stand der Technik bekannt ist.

Jedoch, wie bereits oben erwähnt, sind diese Konstruktionen zur Halterung der Andruckscheibe sehr aufwändig ausgestaltet. Auch sind die Renkverbindungen in der Handhabung bei der DE 20 2010 009 925 U1 relativ ungünstig, da zur manuellen Herstellung dieser Verbindungen die Federscheibe zunächst konzentrisch zum Rahmenelement und in Umfangsrichtung mit ihren Renkverbindungselementen auf die Renkverschlusselemente des Rahmenelements auszurichten ist, um anschließend relativ zum Rahmenelement gedreht werden zu können.

Beim Gegenstand der DE 20 2011 001 772 U1 ist es zusätzlich erforderlich, die Andruckscheibe mit einem radial verlaufenden Griffteil zu versehen, welches zum Wandbereich des Sterilcontainers einen Abstand aufweisen muss, um hintergriffen werden zu können. Mittels dieses Griffteils soll erreicht werden, dass die Andruckscheibe mit den Fingern hintergriffen werden kann, so dass die Rastverbindungen zwischen der Andruckscheibe und den Rastkugeln einfacher gelöst werden können. Auch können sich die Rastverbindungen bei Schlagbeanspruchung auf den Behälterdeckel in ungünstigen Fällen selbständig lösen.

Demgemäß liegt der Erfindung die Aufgabe zugrunde, ausgehend von dem genannten Stand der Technik, eine Filterhalterung für ein Filterblatt derart auszugestalten, dass diese äußerst einfach handhabbar ist und sich sicher nicht selbständig lösen kann.

Die Aufgabe wird erfindungsgemäß zusammen mit den Merkmalen des Oberbegriffs des Anspruches 1 dadurch gelöst, dass auf dem Lagerzapfen ein Haltezapfen mit einer Lagerbohrung aufgesteckt ist, durch welchen die Andruckscheibe gegen den Wandbereich in axialer Richtung pressbar ist,
dass der Haltezapfen in seiner Pressstellung mit dem Lagerzapfen über eine lösbare Formschlussverbindung in Verbindung steht,
dass die Formschlussverbindung mittels einer auf dem Haltezapfen verschiebbar gelagerten Stellhülse sicherbar ist.

Weitere vorteilhafte Ausgestaltungen sind den weiteren Unteransprüchen entnehmbar.

So kann gemäß Anspruch 2 vorgesehen sein, dass die Formschlussverbindung zwischen dem Lagerzapfen und dem Haltezapfen aus einer umlaufenden Rastnut des Lagerzapfens und mehreren jeweils in einer radialen Durchgangbohrung des Haltzapfens angeordneten Haltekugeln besteht, wobei die Haltekugeln in den Durchgangsbohrungen aus einer neutralen nicht mit der Rastnut des Lagerzapfens in Verbindung stehenden radialen Position in eine in die Rastnut eingreifende Sperrposition radial verstellbar ausgebildet sind.

Weiter kann gemäß Anspruch 3 vorgesehen sein, dass die Stellhülse zum Aufschieben auf den Haltezapfen eine Durchgangsbohrung aufweist, welche in ihrem zum Lagerzapfen hin gerichteten Endbereich eine umlaufende, radiale Erweiterung aufweist, welche sich in einer oberen, neutralen Axialposition der Stellhülse auf dem Haltezapfen im Bereich der Haltkugeln befindet und in ihrem Durchmesser derart bemessen ist, dass die Haltkugeln radial in ihre neutrale Position bewegbar sind und, dass bei einer Axialverstellung der Stellhülse auf dem Haltezapfen in ihre Sicherungsposition die im Durchmesser kleinere Durchgangbohrung mit den Haltekugeln derart in Wirkverbindung bringbar ist, dass die Haltekugeln durch die Durchgangsbohrung in ihre in die Lagerbohrung des Haltezapfens hinein ragende und mit der Rastnut des Lagerzapfens eingreifende Sperrposition bringbar sind.

Zur definierten Verstellung der Stellhülse auf dem Haltezapfen kann gemäß Anspruch 4 vorgesehen sein, dass der Haltezapfen in seiner äußeren Mantelfläche eine L-Nut aufweist, welche einen parallel zur Längsmittelachse des Haltzapfens verlaufenden Vertikalabschnitt und einen rechtwinklig dazu verlaufenden Horizontalabschnitt bildet und, dass in der Seitenwand der Stellhülse eine Durchgangsbohrung vorgesehen ist, in welche ein Fixierstift feststehend einsetzbar ist und, dass der Fixierstift im montierten Zustand die Seitenwand radial nach innen durchragt und mit der L-Nut in Eingriff steht und, dass die Stellhülse mit ihrem Fixierstift aus ihrer neutralen oberen Axialposition axial entlang des Vertikalabschnittes in ihre untere Sicherungsposition bringbar und anschließend durch eine Relativdrehung zum Haltezapfen (20) mit ihrem Fixierstift in eine axial unverschiebbare Arretierposition mit dem Horizontalabschnitt in Eingriff bringbar ist. Durch diese Ausgestaltung stehen die Stellhülse und der Haltezapfen des Weiteren unverlierbar miteinander in Verbindung, so dass beim "Anheben" der Stellhülse der Haltezapfen über den mit dessen L-Nut in Verbindung stehenden Fixierstift gleichzeitig mit "angehoben" wird. Dadurch wird die Bedienung erheblich vereinfacht.

Weiter kann gemäß Anspruch 5 vorgesehen sein, dass der Haltezapfen in seinem oberen Endbereich eine durchgehende, quer verlaufende Durchgangsbohrung aufweist, in deren radial äußeren Endbereichen Rastkugeln vorgesehen sind, welche mittels einer Axialdruckfeder radial nach außen gedrückt werden und, dass die Durchgangsbohrung der Stellhülse in ihrem axial oberen Endbereich eine umlaufende Fixiernut aufweist und, dass die axiale Anordnung der Durchgangsbohrung im Haltezapfen und die axial Anordnung der Fixiernut in der Durchgangsbohrung der Stellhülse derart gewählt sind, dass die Rastkugeln mit der Fixiernut in der oberen neutralen Axialposition der Stellhülse miteinander rastend in Eingriff stehen. Durch diese Ausgestaltung wird die obere, neutrale Axialposition der Stellhülse relativ zum Haltezapfen gesichert, so dass ein unbeabsichtigtes Verstellen der Stellhülse in axialer Richtung verhindert wird.

Durch die Ausgestaltung nach Anspruch 6 wird die Arretierposition der Stellhülse auf dem Haltezapfen gesichert. Danach ist vorgesehen, dass die Durchgangsbohrung der Stellhülse axial oberhalb ihrer Fixiernut zwei sich diametral gegenüber liegende Ausfräsungen aufweist und, dass die Umfangspositionen und die axiale Lage der Ausfräsungen der Stellhülse relativ zu den Rastkugeln des Haltezapfens derart gewählt sind, dass die Rastkugeln mit den Ausfräsungen nach der Axialverstellung und Drehung der Stellhülse in ihre Arretierposition rastend miteinander in Eingriff stehen.

Durch die Ausgestaltung nach Anspruch 7 wird erreicht, dass beim Abheben der Stellhülse zusammen mit dem Haltezapfen auch die Andruckscheibe gleichzeitig mit abgehoben wird. Beim Aufsetzen des Haltezapfen auf den Lagerzapfen wird des Weiteren die Andruckscheibe zwangsläufig in ihre korrekte Position auf das Filterblatt bzw. im Bereich der Belüftungsbohrungen aufgesetzt, so dass die Andruckscheibe nicht vorab separat ausgerichtet werden muss. So ist gemäß Anspruch 7 vorgesehen, dass die Andruckscheibe mit dem Haltezapfen feststehend in Verbindung steht.

Durch die Ausgestaltung gemäß Anspruch 8 wird erreicht, dass sich bei einer Relativdrehung der Stellhülse zum Haltezapfen zum Lösen oder Schließen der Formschlussverbindung der Haltezapfen nicht mitdrehen kann, was zu einer sicheren Bedienbarkeit der erfindungsgemäßen Vorrichtung beiträgt. Danach ist vorgesehen, dass der Lagerzapfen in seinem zum Wandabschnitt hin liegenden Endbereich einen radial erweiterten Profilsteg mit gleichmäßig am Umfang verteilt angeordneten Halteflächen aufweist und, dass die Lagerbohrung des Haltezapfens in ihrem zum Wandabschnitt hin liegenden Endbereich ein Lagerprofil aufweist, mit welchem der Haltzapfen in seiner Pressstellung mit den Halteflächen des Profilsteges unverdrehbar in Eingriff steht.

Durch die erfindungsgemäße Ausgestaltung der Filterhaltung, bestehend aus der Andruckscheibe, dem Lagerzapfen, dem Haltezapfen mit seiner Stellhülse und der zugehörigen Formschlussverbindung wird eine äußerst einfache Handhabung beim Austauschen eines Filterblattes erreicht. So ist die komplette Filterhalterung durch eine einfache Dreh- und Axialbewegung in einfacher Weise lösbar und entsprechend in umgekehrter Richtung wieder fixierbar.

Anhand der Zeichnung wird die Erfindung nachfolgend beispielhaft näher erläutert. Es zeigt:
- Fig. 1: eine mögliche Ausführungsvariante eines Sterilcontainers, bestehend aus einem unteren Behälterteil sowie einem Behälterdeckel;
- Fig. 2: eine perspektivische Unteransicht des Behälterdeckels des Sterilcontainers aus Fig. 1;
- Fig. 3: einen perspektivischen Teilschnitt eines Wandbereiches des Behälterdeckels aus Fig. 2 in Unteransicht im Bereich von Belüftungsöffnungen zusammen mit einem Lagerzapfen sowie einer Kontermutter, welche zentral im Bereich der Belüftungsöffnungen feststehend anordenbar sind;
- Fig. 4: eine perspektivische Explosionsdarstellung einer im Teilschnitt dargestellten Andruckscheibe zusammen mit einem Haltezapfen sowie einer Stellhülse;
- Fig. 5: den Haltezapfen aus Fig. 4 in perspektivischer Unteransicht;
- Fig. 6: eine Schnittdarstellung VI-VI der Stellhülse aus Fig. 4;
- Fig. 7: einen perspektivischen Schnitt im zentralen Bereich der Andruckscheibe mit montiertem Haltezapfen sowie aufgesetzter Stellhülse aus Fig. 4;
- Fig. 8: eine perspektivische Schnittdarstellung des Wandabschnittes mit den Belüftungsöffnungen des Behälterdeckels mit montiertem Lagerzapfen sowie auf diesen Lagerzapfen aufgesetzter Andruckscheibe zusammen mit dem feststehend mit der Andruckscheibe in Verbindung stehenden Haltezapfen und aufgesetzter Stellhülse;
- Fig. 9: die Darstellung aus Fig. 8 mit der Stellhülse in ihrer aktiven Sicherungsposition;
- Fig. 10: eine Schnittdarstellung des Wandabschnittes des Behälterdeckels aus den Fig. 1 und 2 im Bereich seiner Belüftungsöffnungen mit arretierter Andruckscheibe.

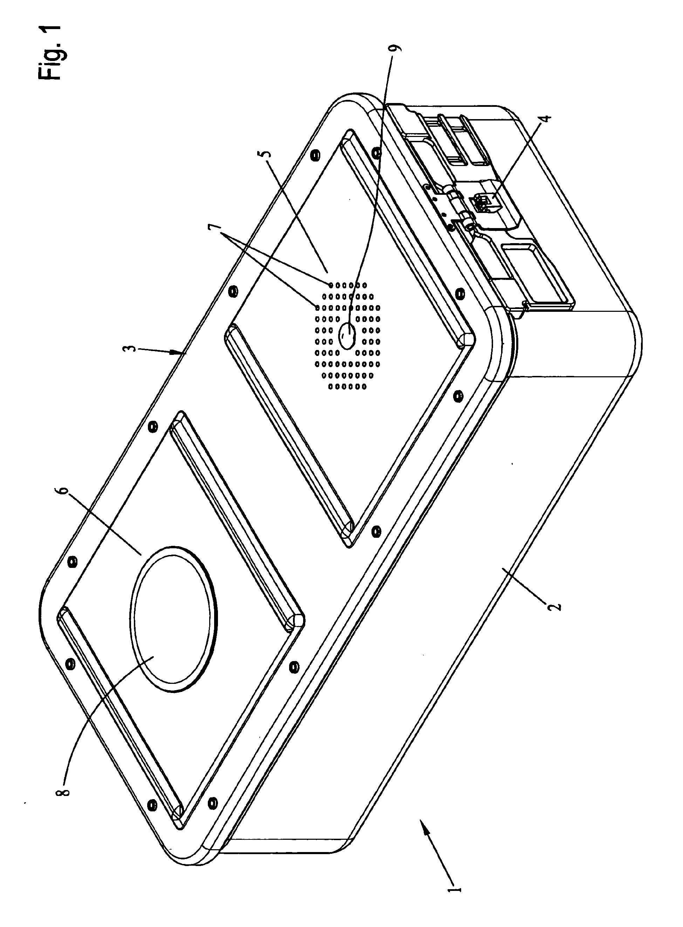

Fig. 1 zeigt beispielhaft einen Sterilcontainer 1, welcher beim vorliegenden Ausführungsbeispiel kastenartig ausgebildet ist und ein unteres Behälterteil 2 aufweist. Auf dieses untere Behälterteil 2 ist ein Behälterdeckel 3 dicht aufgesetzt, welcher in dieser aufgesetzten Position über zwei Behälterverschlüsse 4 gehalten ist, wobei in Fig. 1 lediglich der "vordere" Behälterverschluss 4 sichtbar ist.

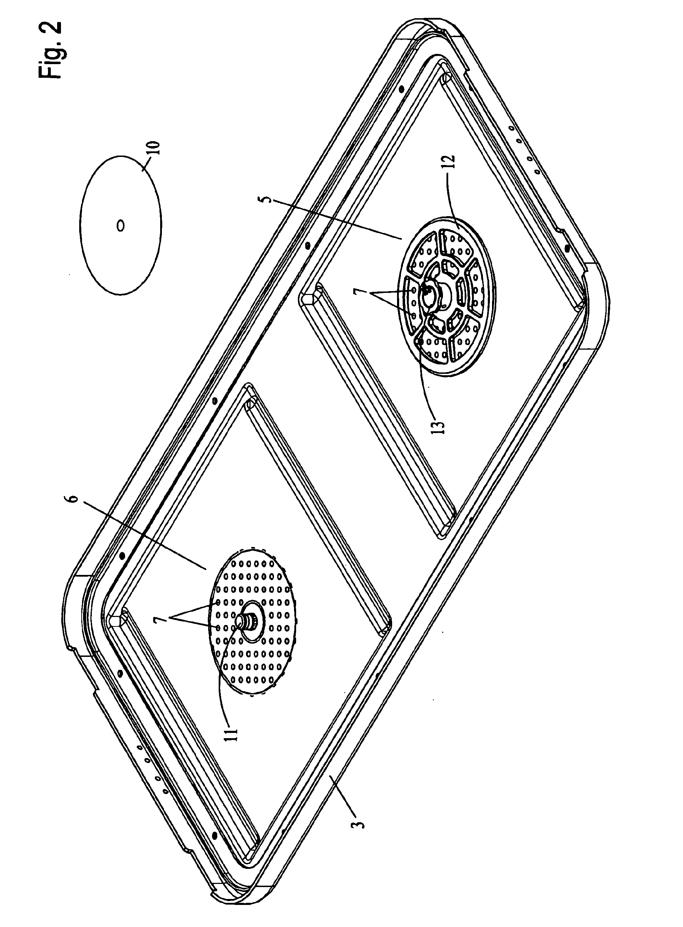

Weiter ist aus Fig. 1 ersichtlich, dass der Behälterdeckel 3 beim vorliegenden Ausführungsbeispiel zwei Wandbereiche 5 und 6 aufweist, welche jeweils mit mehreren Belüftungsöffnungen 7 versehen sind. Die Belüftungsöffnungen 7 des Wandbereiches 6 sind in der Unteransicht der Fig. 2 dargestellt.

Die beiden Wandbereiche 5 und 6 mit ihren Belüftungsöffnungen 7 unterscheiden sich dadurch, dass die Belüftungsöffnungen im zweiten Wandbereich 6 durch eine Abdeckplatte 8 abgedeckt sind. Diese Abdeckplatte 8 weist zum Wandbereich 6 einen vertikalen Abstand auf, so dass die aus Fig. 2 ersichtlichen Belüftungsöffnungen 7 mit Umgebungsluft durchströmt werden können.

Des Weiteren ist aus Fig. 1 ersichtlich, dass zentral innerhalb der Belüftungsöffnungen 7 des Wandbereiches 5 eine flache Kontermutter 9 vorgesehen ist, deren Bedeutung später noch näher erläutert wird.

Die Belüftungsöffnungen 7 dienen dem "Druckausgleich" innerhalb des geschlossenen Sterilcontainers 1 während des Betriebes. So dient ein solcher Sterilcontainer 1, wie bereits in der Beschreibungseinleitung erwähnt, beispielsweise zur Sterilisation von chirurgischen Instrumenten. Zu diesem Zweck werden die chirurgischen Instrumente in den Sterilcontainer 1 eingebracht, der Behälterdeckel 3 auf das Behälterteil 2 aufgesetzt und dicht verschlossen. Beim Sterilprozess wird der Sterilcontainer 1 in der Regel erwärmt, so dass sich innerhalb dieses Sterilcontainers 1 ein Überdruck bildet. Zum Druckausgleich durchströmt nun die Luft die Belüftungsöffnungen 7 von innen nach außen.

Nach dem Sterilisationsvorgang wird nunmehr der Sterilcontainer 1 wieder abgekühlt, so dass in entgegengesetzter Richtung Umgebungsluft durch die Belüftungsöffnungen 7 in den Sterilcontainer 1 hineinströmt. Um aus hygienischen Gründen Verunreinigungen innerhalb des Behälters bzw. der sterilisierten medizinischen Instrumente zu verhindern, ist eine sogenannte Sterilbarriere vorgesehen, welche bei der dargestellten Ausführungsvariante in Form eines Filterblattes 10 ausgeführt ist, welches in Fig. 2 als separates Bauteil dargestellt ist.

Wie weiter aus der Unteransicht des Behälterdeckels 3 aus Fig. 2 ersichtlich ist, ist im Wandbereich 6 bzw. im Bereich der Belüftungsöffnungen 7, zentral ein Lagerzapfen 11 vorgesehen, welcher in eine entsprechende, in Fig. 2 nicht sichtbare, Gewindebohrung des Wandbereiches 6 des Behälterdeckels 3 eingeschraubt ist. Zur Sicherung dieses Lagerzapfens 11 dient im Bereich des Behälterdeckels 3 die Abdeckplatte 8, welche auf einen Gewindeabschnitt, welcher oberseitig über den Wandabschnitt 6 des Behälterdeckels 3 hinausragt, aufgeschraubt ist.

Nachdem der Lagerzapfen 11 positioniert ist, kann nunmehr das Filterblatt 10 auf diesen im Bereich der Belüftungsöffnungen 7 aufgesetzt werden. Anschließend, wie dies für den Wandbereich 5 in Fig. 2 dargestellt ist, wird auf das Filterblatt 10 eine Andruckscheibe 12 aufgesetzt, welche beim dargestellten Ausführungsbeispiel über eine spezielle Haltevorrichtung 13 gegen das Filterblatt 10 oberseitig verspannt wird. Somit bildet die Haltevorrichtung 13 zusammen mit der Andruckscheibe 12 eine Filterhalterung zur feststehenden Halterung des Filterblattes 10 im Bereich der Belüftungsöffnungen 7.

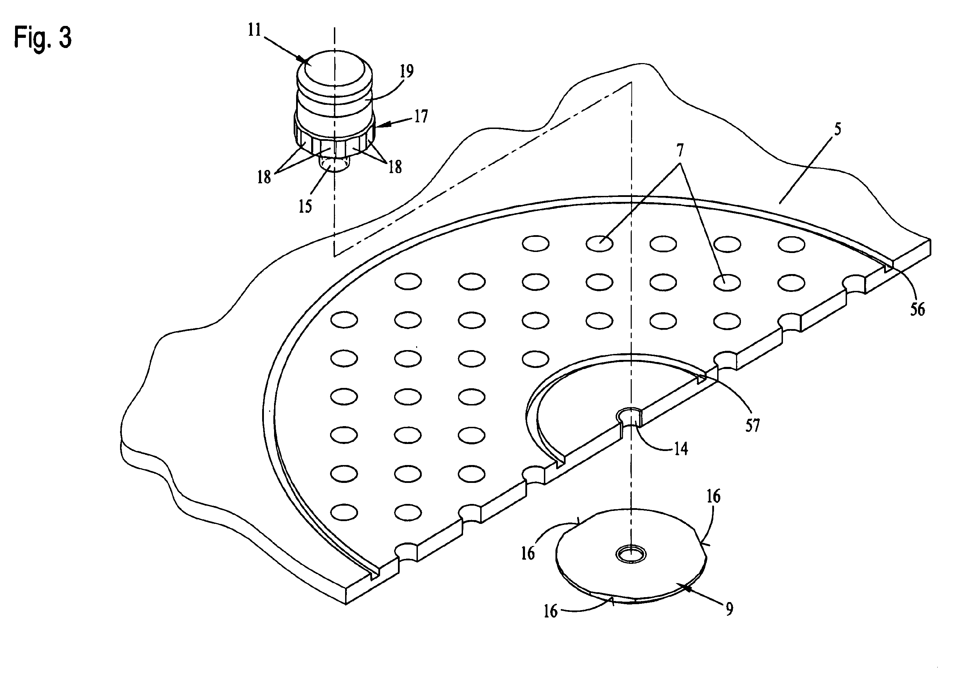

Hierzu zeigt zunächst Fig. 3 einen Teilschnitt des Wandbereiches 5 mit den Belüftungsöffnungen 7. Es ist erkennbar, dass zentral im Bereich der Belüftungsöffnungen 7 ein Innengewinde 14 vorgesehen ist, in welches ein Gewindezapfen 15 des Lagerzapfens 11 einschraubbar ist. Die axiale Länge dieses Gewindezapfens 15 ist dabei derart gewählt, dass dieser Gewindezapfen im montierten Zustand durch den Wandbereich 5 hindurchragt. Dementsprechend kann die Kontermutter 9 auf diesen Gewindezapfen 15 aufgeschraubt werden, so dass der Lagerzapfen 11 festsitzend im Wandbereich 5 angeordnet ist. Um die Kontermutter 9 festsitzend auf den Gewindezapfen 15 aufschrauben zu können, weist diese bei dem dargestellten Ausführungsbeispiel insgesamt drei gleichmäßig am Umfang verteilt angeordnete, ebene Schlüsselflächen 16 auf.

Weiter ist aus Fig. 3 erkennbar, dass der Lagerzapfen 11 in seinem zum Gewindezapfen 15 hin liegenden Endbereich einen radial erweiterten Profilsteg 17 aufweist, welcher mehrere, eben ausgebildete und gleichmäßig am Umfang verteilt angeordnete Halteflächen 18 bildet. Im gegenüberliegenden Endbereich dieses Profilsteges 17 weist der Lagerzapfen 11 eine umlaufende Rastnut 19 auf, welche eine etwa teilzylindrisch ausgebildete Querschnittsform hat.

Wie bereits zu Fig. 2 erwähnt, ist auf diesen Lagerzapfen 11 im montierten Zustand die Haltevorrichtung 13 lösbar aufsetzbar.

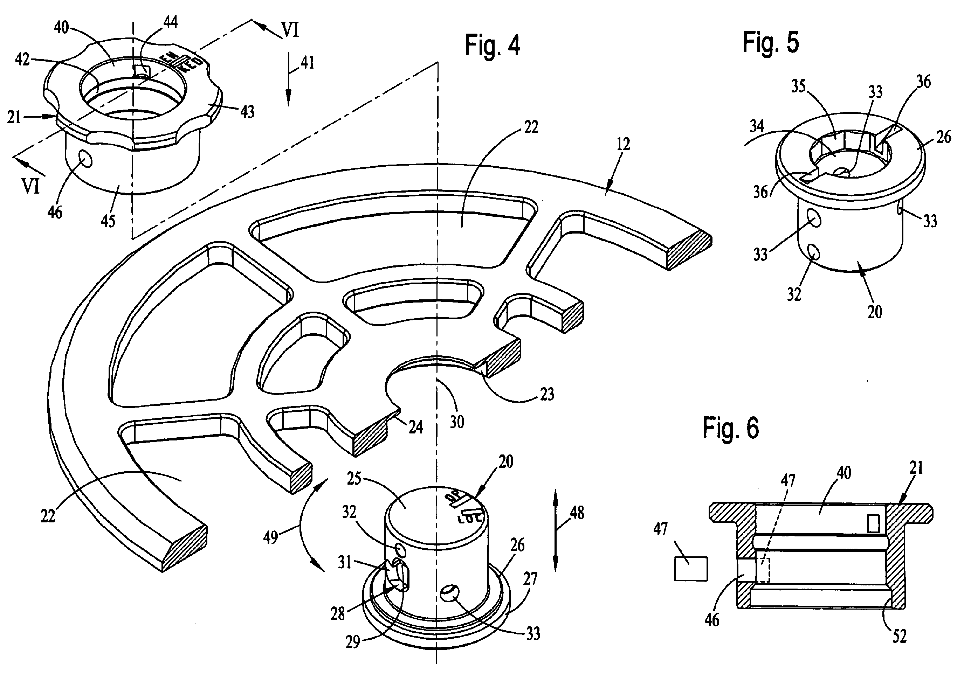

Hierzu zeigt Fig. 4 einen perspektivischen Teilschnitt der Andruckscheibe 12 aus Fig. 2 zusammen mit einem Haltezapfen 20 und einer Stellhülse 21. Dieser Haltezapfen 20 und die Stellhülse 21 bilden gemeinsam die zu Fig. 2 erwähnte Haltevorrichtung 13 für die Andruckscheibe 12. Die Andruckscheibe 12 weist mehrere Durchbrüche 22 auf, so dass diese entsprechend mit einer Luftströmung durchströmt werden kann. Zentral ist die Andruckscheibe 12 mit einem Innengewinde 23 versehen, welches oberseitig mittels eines radial nach innen vorstehenden Anschlagsteges 24 begrenzt ist.

Entsprechend dem Durchmesser dieses Anschlagsteges 24 weist der Haltezapfen 20 einen vertikal nach oben vorstehenden zylindrischen Lagerabschnitt 25 auf.

Im unteren Endbereich dieses Lagerabschnittes 25 bildet der Haltezapfen 20 einen radial vorstehenden, umlaufenden Montagesteg 26, welcher mit einem entsprechenden Außengewinde 27 versehen ist (in der Zeichnung nicht explizit dargestellt). Zur Montage in der Andruckscheibe 12 ist der Haltezapfen 20 mit seinem zylindrischen Lagerabschnitt 25 durch den Anschlagsteg 24 hindurch steckbar und kann mit seinem Außengewinde 27 mit dem Innengewinde 23 der Andruckscheibe 12 feststehend in Eingriff gebracht werden.

Weiter ist aus Fig. 4 ersichtlich, dass der zylindrische Lagerabschnitt 25 des Haltezapfens 20 in seiner Mantelfläche im vorderen linken Bereich eine L-förmige L-Nut 28 aufweist, welche zum oberen Endbereich hin einen Vertikalabschnitt 29 aufweist, welcher sich parallel zur Längsmittelachse 30 des Lagerabschnittes 25 bzw. des Haltezapfens 20 erstreckt. Im unteren Endbereich dieses Vertikalabschnittes 29 bildet die L-Nut 28 einen quer verlaufenden Horizontalabschnitt 31.

Weiter ist aus Fig. 4 und auch aus der Unteransicht des Lagerzapfens 20 aus Fig. 5 erkennbar, dass im freien Endbereich des Lagerabschnittes 25 eine Durchgangsbohrung 32 vorgesehen ist. Diese Durchgangsbohrung 32 dient zur Aufnahme zweier Rastkugeln, zwischen welchen eine Axialdruckfeder vorgesehen ist, wie später noch näher erläutert wird.

Im zum Montagesteg 26 hin liegenden Bereich weist der Lagerabschnitt 25 insgesamt drei Aufnahmebohrungen 33 auf (Fig. 5), welche jeweils zur Aufnahme eines Feststellelementes in Form einer Haltekugel dienen, wie ebenfalls später noch näher erläutert wird.

Weiter ist aus Fig. 5 ersichtlich, dass der Haltezapfen 20 eine zentrale Lagerbohrung 34 aufweist, mit welcher dieser Haltezapfen 20 passend auf den Lagerzapfen 11 aus Fig. 3 aufsetzbar ist. Des Weiteren ist diese Lagerbohrung 34 im axialen Endbereich des Montagesteges 26 mit einem Lagerprofil 35 versehen, mit welchem der Haltezapfen 20 passend auf die Halteflächen 18 des Profilsteges 17 des Lagerzapfens 11 aufsetzbar ist. Damit ist der Haltezapfen 20 im montierten Zustand gegen Drehung gesichert auf dem Lagerzapfen 11 anordenbar.

Weiter ist aus Fig. 5 noch ersichtlich, dass im Bereich des Montagesteges 26 unterseitig in dessen Stirnfläche zwei Nuten 36 angeordnet sind, welche zum feststehenden Anziehen des Haltezapfens 20 im Innengewinde 23 der Andruckscheibe 12 dienen.

Weiter ist aus Fig. 4 ersichtlich, dass die Stellhülse 21 eine zentrale Durchgangsbohrung 40 bildet, welche in axialem Abstand zu ihrer oberen Stirnfläche in Richtung des Pfeiles 41 eine innere Fixiernut 42 aufweist.

Im oberen Endbereich weist die Stellhülse 21 einen radial erweiterten, profilierten Betätigungssteg 43 auf, welcher zur vereinfachten Bedienung der Stellhülse 21 dient. Im Bereich zwischen der inneren Fixiernut 42 und der oberen Stirnfläche bzw. im Bereich dieses Betätigungssteges 43, ist die Durchgangsbohrung 40 mit einer Ausfräsung 44 versehen, welcher diametral gegenüberliegend eine zweite Ausfräsung 44 angeordnet sein kann, welche in Fig. 4 nicht erkennbar ist.

Im Betrieb dient diese Ausfräsung bzw. dienen diese Ausfräsungen 44 zur Fixierung einer vorgegebenen, relativen Winkelstellung der Stellhülse 21 zum Haltezapfen 20. Dabei gelangen in dieser Stellung die Ausfräsungen 44 mit der Durchgangsbohrung 32 in Überdeckung, so dass die in der Durchgangsbohrung 32 unter Federbelastung angeordneten Rastkugeln mit diesen Ausfräsungen 44 in Eingriff gelangen.

Weiter ist aus Fig. 4 ersichtlich, dass die Stellhülse 21 in ihrer zylindrischen Seitenwand 45 eine Durchgangsbohrung 46 aufweist.

Wie aus der Schnittdarstellung aus Fig. 6 der Stellhülse 21 ersichtlich ist, kann mit dieser Durchgangsbohrung 46 ein Fixierstift 47 in Eingriff gebracht werden, welcher im montierten Zustand feststehend in der Durchgangsbohrung 46 sitzt und, wie dies in Fig. 6 in gestrichelten Linien dargestellt ist, in die Durchgangsbohrung 46 hineinragt. Dieser Fixierstift 47 wird nach dem Aufsetzen der Stellhülse 21 auf den Haltezapfen 20 eingesetzt und greift im montierten Zustand in die L-Nut 28 bzw. deren Vertikalabschnitt 29 bzw. Horizontalabschnitt 31 wahlweise ein. Damit wird erreicht, dass die Stellhülse 21 zum einen auf den Haltezapfen 20 begrenzt in axialer Richtung des Doppelpfeils 48 verstellbar und ebenfalls begrenzt in Richtung des Doppelpfeils 49 drehbar ist. Der Fixierstift 47 kann in die Durchgangsbohrung 46 eingepresst, verlötet oder geklebt sein. Auch vorstellbar ist eine "klemmende" Gewindeverbindung, so dass sich der Fixierstift 47 nicht selbständig in der Durchgangbohrung, welche in diesem Fall als Gewindebohrung ausgebildet ist, lösen kann.

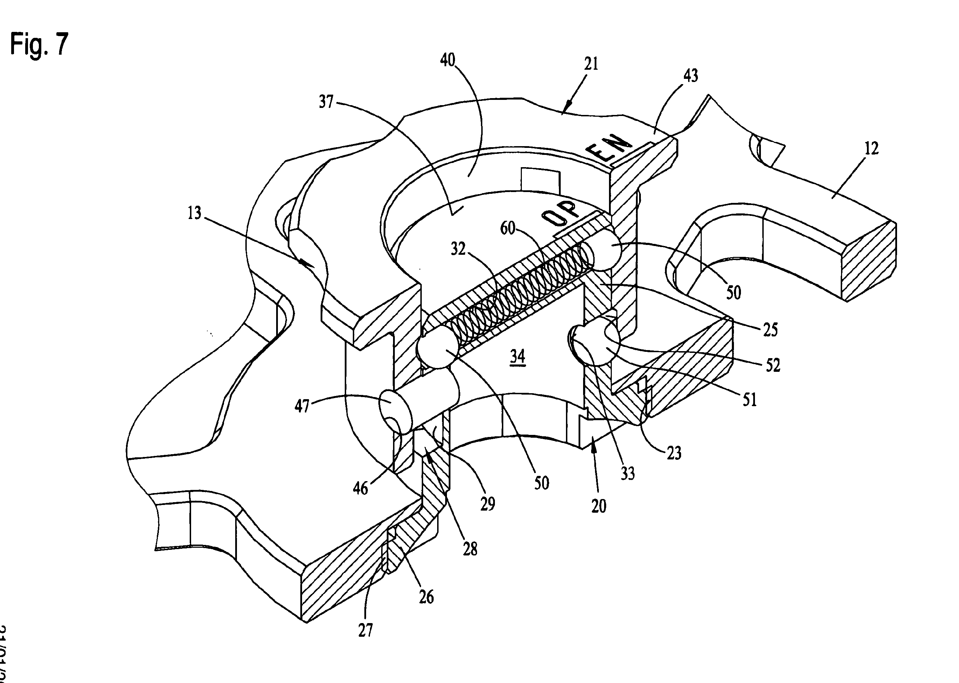

Bezüglich des vormontierten Zustandes der Andruckscheibe 12, des Haltezapfens 20 und der Stellhülse 21 zeigt Fig. 7 einen vergrößerten Teilschnitt dieser Bauteile im zentralen Bereich der Andruckscheibe 12.

Aus Fig. 7 ist erkennbar, dass die Stellhülse 21 mit ihrer Durchgangsbohrung 40 auf den zylindrischen Lagerabschnitt 25 des Halterzapfens 20 aufgesetzt ist. Dabei befindet sich die Stellhülse 21 mit ihrem Betätigungssteg 43 oberhalb der oberen Stirnfläche 37 des zylindrischen Lagerabschnittes 25. In dieser "neutralen" Position der Stellhülse 21 relativ zum Haltezapfen 20 greifen die beiden oben erwähnten und in Fig. 7 dargestellten Rastkugeln 50 in die umlaufende Fixiernut 42 der Stellhülse 21 ein. Damit ist die neutrale, axiale Relativstellung der Stellhülse 21 zum Haltezapfen 20 gesichert.

Weiter ist aus Fig. 7 erkennbar, dass der Fixierstift 47 festsitzend in der Durchgangsbohrung 46 angeordnet ist und mit dem Vertikalabschnitt 29 der L-Nut 28 in deren oberen Endbereich in Eingriff steht. In dieser Position wird somit die Stellhülse 21 gegen Verdrehen gegen den Haltezapfen 20 aufgrund der Wirkverbindung des Fixierstiftes 47 mit dem Vertikalabschnitt 29 der L-Nut 28 fixiert.

Weiter ist aus Fig. 7 noch eine der drei radialen Durchgangsbohrungen 33 des Lagerabschnittes 25 aus Fig. 5 erkennbar, in welcher eine entsprechende Haltekugel 51 angeordnet ist. Dementsprechend sind auch in den zwei weiteren in Fig. 7 nicht erkennbaren Durchgangsbohrungen weitere Haltekugeln 51 eingesetzt.

In dieser axialen Relativstellung der Stellhülse 21 zum Haltezapfen 20 befindet sich diese Haltekugel 51 (bzw. die Rastkugeln) in einem axialen Bereich einer radialen Erweiterung 52 der Durchgangsbohrung 40 der Stellhülse 21, welche im unteren Endbereich der Stellhülse 21 angeordnet ist, wie dies insbesondere auch aus der Schnittdarstellung der Fig. 6 erkennbar ist. Insoweit kann die Haltekugel 51 (bzw. können die Haltekugeln) radial soweit nach außen bewegt werden, bis diese an dieser radialen Erweiterung 52 innen anliegt und nicht mehr in die Lagerbohrung 34 des Haltezapfens 20 hineinragt.

Des Weiteren ist aus Fig. 7 noch erkennbar, dass der Haltezapfen 20 mit seinem Außengewinde 27 seines Montagesteges 26 mit dem Innengewinde 23 der Andruckscheibe 12 feststehend in Eingriff steht.

In diesem vormontierten Zustand ist die Andruckscheibe 12 mit der aus dem Haltezapfen 20 und der Stellhülse 21 bestehenden Haltevorrichtung 13 auf den Lagerzapfen 11 nach Fig. 3 aufsetzbar. Des Weiteren ist aus Fig. 7 noch die Axialdruckfeder 60 erkennbar, durch welche die beiden in den Endbereich der quer verlaufenden Durchgangbohrung 32 angeordneten Rastkugeln 50 unter Vorspannung radial nach außen gedrückt werden.

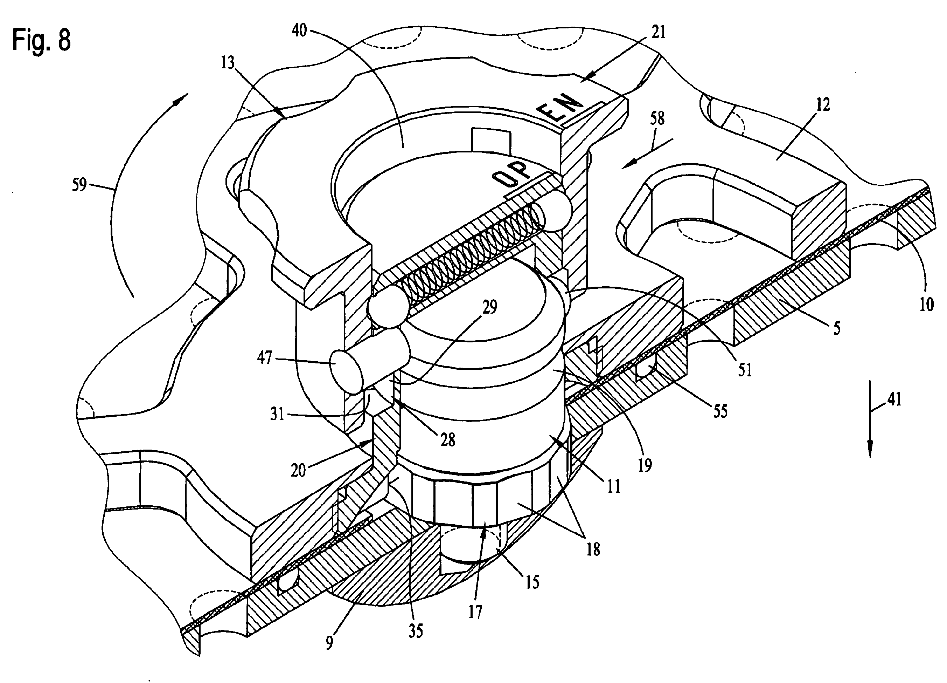

Fig. 8 zeigt die Darstellung aus Fig. 7 mit der Andruckscheibe 12 der Haltevorrichtung 13 in ihrem auf den Lagerzapfen 11 aufgesetzten Zustand. Der Lagerzapfen 11 ist in den Wandabschnitt 5 eingeschraubt, wobei unterseitig auf den Gewindezapfen 15 des Lagerzapfens 11 die Kontermutter 9 feststehend aufgeschraubt ist. In diesem montierten Zustand des Lagerzapfens 11 im Wandbereich 5 ist dieser unverdrehbar auf dem Wandbereich 5 fixiert.

Des Weiteren ist erkennbar, dass in diesem "lose" aufgesetzten Zustand der Andruckscheibe 12 mit der Haltevorrichtung 13 auf den Lagerzapfen 11, sich die Haltekugel 51 im axialen Bereich der umlaufenden Rastnut 19 des Lagerzapfens 11 befindet. Der Haltezapfen 20 steht in diesem aufgesetzten Zustand mit seinem, in Fig. 8 nur andeutungsweise erkennbaren Lagerprofil 35, formschlüssig und damit unverdrehbar mit den Halteflächen 18 des Profilsteges 17 des Lagerzapfens 11 in Eingriff. Damit wird auch die Andruckscheibe 12 aufgrund der feststehenden Gewindeverbindung mit dem Haltezapfen 20 unverdrehbar im Wandbereich 5 gehalten.

Weiter ist aus Fig. 8 ersichtlich, dass zwischen der Andruckscheibe 12 und dem Wandbereich 5, das in Fig. 2 als separates Bauteil dargestellte Filterblatt 10 angeordnet ist. Des Weiteren ist aus Fig. 8 noch erkennbar, dass unterhalb des Filterblattes 10 ein Dichtungsring 55 vorgesehen ist.

Hierzu sei noch kurz auf die Darstellung der Fig. 3 verwiesen, aus welcher erkennbar ist, dass im äußeren Umfangsbereich der Belüftungsbohrungen 7 eine Aufnahmenut 56 angeordnet ist, in welcher im montierten Zustand ein zweiter Dichtungsring vorgesehen ist, welcher in Fig. 3 nicht explizit dargestellt ist. Zur Aufnahme des Dichtungsringes 55 aus Fig. 8 weist der Wandbereich 5 im radial innerhalb der Belüftungsbohrungen 7 liegenden Bereich ebenfalls eine umlaufende Aufnahmenut 57 auf.

Um nun die Andruckscheibe 12 zumindest leicht gegen die Oberfläche des Filterblattes 10 zu pressen und insbesondere eine Dichtwirkung des Dichtungsringes 55 zu erreichen, ist nunmehr die Stellhülse 21 in Richtung des Pfeiles 41 manuell nach unten verstellbar. Dabei gelangt die Haltekugel 51 (bzw. die Haltekugeln) aus dem Bereich der radialen Erweiterung 52 der Durchgangsbohrung 40 in die radial verjüngt ausgebildete Durchgangsbohrung 40, so dass eine Stellbewegung in radialer Richtung des Pfeiles 58 der Haltekugel 51 bewirkt wird. Damit wird die Haltekugel 51 zwangsläufig in die umlaufende Rastnut 19 gepresst, wodurch wiederum Stellkräfte in Richtung des Pfeiles 41 bewirkt werden. Da insgesamt drei solcher Haltekugeln 51 in den in Fig. 5 erkennbaren Durchgangsbohrungen 33 vorgesehen sind, wird somit ein gleichmäßiges Anpressen der Andruckscheibe 12 über diese Haltekugeln 51 und die Rastnut 19 in Richtung des Pfeiles 41 bewirkt.

Bei dieser Stellbewegung in Richtung des Pfeiles 41 "gleitet" der Fixierstift 47 entlang des Vertikalabschnittes 29 der L-Nut 28. Mit Erreichen der "unteren" Endstellung der Stellhülse 21 befindet sich dieser Fixierstift 47 im axialen Bereich des unteren Horizontalabschnittes 31 der L-Nut 28. So kann nun zur Fixierung dieser axialen Sicherungsposition der Stellhülse 21 durch Drehen derselben in Richtung des Pfeiles 59 in ihre Fixierposition der Fixierstift 47 mit dem Horizontalabschnitt 31 in Eingriff gebracht werden.

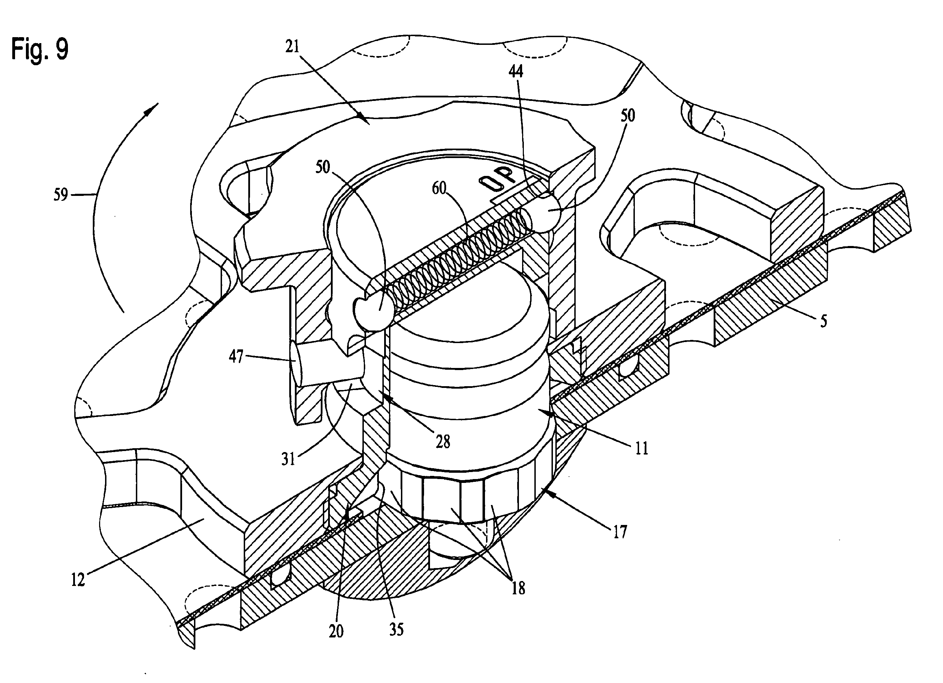

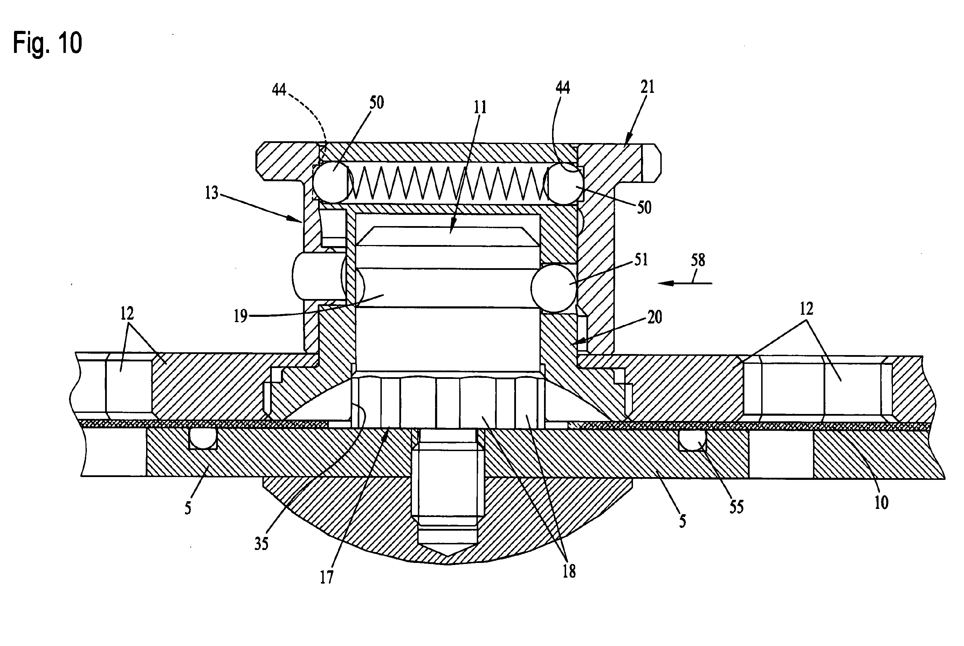

Diese fixierte Fixierposition der Stellhülse 21 mit ihrem Fixierstift 47 ist in der perspektivischen Schnittdarstellung der Fig. 9 erkennbar. Auch erkennbar ist in dieser Darstellung, dass die Rastkugel 50 mit der Ausfräsung 44 des Durchgangsbohrung 40 der Stellhülse 21 in Eingriff steht und in dieser Eingriffsposition durch die Axialdruckfeder 60 gehalten ist. Die zweite Rastkugel 50 steht dementsprechend mit der zweiten, in Fig. 9 nicht erkennbaren Ausfräsung in Eingriff. Diese Eingriffsposition der zweiten Rastkugel 50 mit der zweiten Ausfräsung 44 ist aus Fig. 10 erkennbar, wobei die zweite Ausfräsung 44 in Fig. 10 in gestrichelten Linen dargestellt ist, da diese auf Grund der Schnittführung aus Fig. 9 in Fig. 10 "eigentlich" nicht erkennbar ist, da diese "vor" der Ebene des Zeichnungsblattes liegt.

Wie aus Fig. 9 weiter ersichtlich ist, steht der Fixierstift 47 mit dem Horizontalabschnitt 31 der L-Nut 28 in Eingriff. Aufgrund der formschlüssigen Verbindung des Lagerzapfens 11 mit den Halteflächen 18 des Profilsteges 17 mit dem inneren Lagerprofil 35 des Haltezapfens 20 ist der Haltezapfen 20 ebenfalls unverdrehbar relativ zum Wandabschnitt 5 auf dem Lagerzapfen 11 fixiert. Insoweit ist sichergestellt, dass sich bei der drehenden Stellbewegung der Stellhülse 21 in Richtung des Pfeiles 59 der Haltezapfen 20 nicht mitdrehen kann.

Aufgrund des Eingriffs der beiden Rastkugeln 50 in die zugehörigen Ausfräsungen 44 der Stellhülse 21 wird die Stellhülse 21 in dieser "arretierten" Position gesichert.

Weiter ist aus Fig. 10 ersichtlich, dass die Haltekugel 51, von welchen drei Stück gleichmäßig am Umfang verteilt angeordnet sind, mit der Rastnut 19 des Lagerzapfens 11 in Eingriff steht. Dabei stützt sich diese Haltekugel 51 an der Innenwand der Durchgangsbohrung 40 der Stellhülse 21 ab, so dass die arretierte Position des Haltezapfens 20 zusammen mit der Andruckscheibe 12 sicher fixiert ist. In dieser fixierten Position wird beispielsweise der Dichtungsring 55 im Wandbereich 5 zumindest leicht zusammengepresst, so dass hier eine optimale Dichtwirkung erreichbar ist, wie dies aus Fig. 10 erkennbar ist.

Durch entsprechend entgegengesetzte Drehbewegung entgegen dem Pfeil 59 aus der in den Fig. 10 und 9 dargestellten Arretierposition und anschließendes Anheben der Stellhülse 21 entgegen dem Pfeil 41 gelangt die Stellhülse 21 mit ihrer radialen Erweiterung 52 wiederum in den Axialbereich der Haltekugeln 51, so dass diese entgegen dem Pfeil 58 radial nach außen verstellbar sind, bis diese an der Innenwand der radialen Erweiterung 52 zur Anlage kommen. Dabei wird der axiale Stellweg der Stellhülse 21 zum Haltezapfen 20 entgegen dem Pfeil 41 durch die axiale Länge des Vertikalabschnittes 29 der L-Nut 28 begrenzt. Durch die radiale Stellbewegung der Haltekugeln 51 wird die Formschlussverbindung zwischen diesen Haltekugeln 51 und der Rastnut 19 des Lagerzapfens 11 aufgehoben, so dass die gesamte Haltevorrichtung 13 zusammen mit der mit dieser feststehend in Verbindung stehenden Abdeckscheibe 12 abgehoben und das darunter liegende Filterblatt 10 ausgetauscht werden kann.

Es ist leicht vorstellbar, dass aufgrund der speziellen Ausgestaltung, insbesondere der Haltevorrichtung 13, die Andruckscheibe 12 in äußerst einfacher Weise montiert und demontiert werden kann. Zur Montage wird letztendlich die Andruckscheibe 12 zusammen mit der Haltevorrichtung 13 einfach auf den Lagerzapfen 11 aufgesteckt, bis die Halteflächen 18 des Profilsteges 17 formschlüssig mit dem Lagerprofil 35 des Haltezapfens 20 in Eingriff stehen. Da nunmehr eine drehfeste Verbindung dieses Haltezapfens 20 mit dem Lagerzapfen 11 erreicht ist, ist somit die Andruckscheibe 12 ebenfalls unverdrehbar zum Wandabschnitt 5 auf diesem angeordnet. Durch anschließendes Verstellen der Stellhülse 21 in axialer Richtung des Pfeiles 41 und anschließendes Drehen in Richtung des Pfeiles 59 kann diese Position in einfachster Art und Weise fixiert werden. In entsprechender Umkehrung dieses Bewegungsablaufes ist die Andruckscheibe 12 wiederum in einfachster Weise vom Filterblatt 10 abhebbar, so dass dieses einfach austauschbar ist.

Aufgrund der Fixierung der Andruckscheibe 12 über die Halteflächen 18 des Profilsteges 17 des Lagerzapfens 11 und das Lagerprofil 35 des Haltezapfens 20 ist die Andruckscheibe 12 unverdrehbar relativ zum Wandabschnitt 5 und damit zum Filterblatt 10 fixiert, so dass eine Beschädigung, insbesondere bei der Montage des Filterblattes 10 sicher ausgeschlossen ist.

## Patentansprüche

1. Filterhalterung mit einem Sterilcontainer (1), welcher in einem Wandbereich (5) oder in mehreren Wandbereichen (5, 6) mit Belüftungsöffnungen (7) versehen ist, durch welche im geschlossenen Zustand ein Luftaustausch zwischen dem Innenraum des Sterilcontainers (1) und der Umgebung stattfindet, wobei die Belüftungsöffnungen (7) mittels eines Filterblattes (10) zur Bildung einer Sterilbarriere abgedeckt sind, welche eine Sterilisation der durch die Belüftungsöffnungen in den Sterilraum gelangenden Luft bewirkt, wobei das Filterblatt (10) durch eine luftdurchlässige Andruckscheibe (12) auf dem Wandbereich (5, 6) gehalten ist,
wobei im Wandbereich (5, 6) der Belüftungsöffnungen (7) ein über den Wandbereich (5, 6) vorstehender, feststehend mit dem Wandbereich (5, 6) in Verbindung stehender Lagerzapfen (11) vorgesehen ist,
**dadurch gekennzeichnet,**
**dass** auf dem Lagerzapfen (11) ein Haltezapfen (20) mit einer Lagerbohrung (34) aufgesteckt ist, durch welchen die Andruckscheibe (12) gegen den Wandbereich (5, 6) in axialer Richtung (41) pressbar ist,
**dass** der Haltezapfen (20) in seiner Pressstellung mit dem Lagerzapfen (11) über eine lösbare Formschlussverbindung (19, 51) in Verbindung steht,
**dass** die Formschlussverbindung (19, 51) mittels einer auf dem Haltezapfen (20) verschiebbar gelagerten Stellhülse (21) sicherbar ist.

2. Filterhalterung nach Anspruch 1, **dadurch gekennzeichnet, dass** die Formschlussverbindung zwischen dem Lagerzapfen (11) und dem Haltezapfen (20) aus einer umlaufenden Rastnut (19) des Lagerzapfens (11) und mehreren jeweils in einer radialen Durchgangbohrung (33) des Haltzapfens (20) angeordneten Haltekugeln (51) besteht, wobei die Haltekugeln (51) in den Durchgangsbohrungen (33) aus einer neutralen nicht mit der Rastnut (19) des Lagerzapfens (11) in Verbindung stehenden radialen Position in eine in die Rastnut (19) eingreifende Sperrposition radial verstellbar ausgebildet sind.

3. Filterhalterung nach Anspruch 2, **dadurch gekennzeichnet, dass** die Stellhülse (21) zum Aufschieben auf den Haltezapfen (20) eine Durchgangsbohrung (40) aufweist, welche in ihrem zum Lagerzapfen (11) hin gerichteten Endbereich eine umlaufende, radiale Erweiterung (52) aufweist, welche sich in einer oberen, neutralen Axialposition der Stellhülse (21) auf dem Haltezapfen (20) im Bereich der Haltkugeln (51) befindet und in ihrem Durchmesser derart bemessen ist, dass die Haltkugeln (51) radial in ihre neutrale Position bewegbar sind und,
dass bei einer Axialverstellung (41) der Stellhülse (21) auf dem Haltezapfen (20) in ihre Sicherungsposition die im Durchmesser kleinere Durchgangbohrung (40) mit den Haltekugeln (51) derart in Wirkverbindung bringbar ist, dass die Haltekugeln (51) durch die Durchgangsbohrung (40) in ihre in die Lagerbohrung (34) des Haltezapfens (20) hinein ragende und mit der Rastnut (19) des Lagerzapfens (11) eingreifende Sperrposition bringbar sind.

4. Filterhalterung nach Anspruch 2 bis 3, **dadurch gekennzeichnet, dass** der Haltezapfen (20) in seiner äußeren Mantelfläche eine L-Nut (28) aufweist, welche einen parallel zur Längsmittelachse (30) des Haltzapfens (20) verlaufenden Vertikalabschnitt (29) und einen rechtwinklig dazu verlaufenden Horizontalabschnitt (31) bildet und,
dass in der Seitenwand (45) der Stellhülse (21) eine Durchgangsbohrung (46) vorgesehen ist, in welche ein Fixierstift (47) feststehend einsetzbar ist und,
dass der Fixierstift (47) im montierten Zustand die Seitenwand (45) radial nach innen durchragt und mit der L-Nut (28) in Eingriff steht und,
dass die Stellhülse (21) mit ihrem Fixierstift (47) aus ihrer neutralen oberen Axialposition axial (41) entlang des Vertikalabschnittes (29) in ihre untere Sicherungsposition bringbar und anschließend durch eine Relativdrehung (59) zum Haltezapfen (20) mit ihrem Fixierstift (47) in eine axial unverschiebbare Arretierposition mit dem Horizontalabschnitt (31) in Eingriff bringbar ist.

5. Filterhalterung nach Anspruch 3 oder 4, **dadurch gekennzeichnet, dass** der Haltezapfen (20) in seinem oberen Endbereich eine durchgehende, quer verlaufende Durchgangsbohrung (32) aufweist, in deren radial äußeren Endbereichen Rastkugeln (50) vorgesehen sind, welche mittels einer Axialdruckfeder (60) radial nach außen gedrückt werden und,
dass die Durchgangsbohrung (40) der Stellhülse (21) in ihrem axial oberen Endbereich eine umlaufende Fixiernut (42) aufweist und,
dass die axiale Anordnung der Durchgangsbohrung (32) im Haltezapfen (29) und die axiale Anordnung der Fixiernut (42) in der Durchgangsbohrung (40) der Stellhülse (21) derart gewählt sind, dass die Rastkugeln (50) mit der Fixiernut (42) in der oberen neutralen Axialposition der Stellhülse (21) miteinander rastend in Eingriff stehen.

6. Filterhalterung nach Anspruch 5, **dadurch gekennzeichnet, dass** die Durchgangsbohrung (40) der Stellhülse (21) axial oberhalb ihrer Fixiernut (42) zwei sich diametral gegenüber liegende Ausfräsungen (44) aufweist und,
dass die Umfangspositionen und die axiale Lage der Ausfräsungen (44) der Stellhülse (21) relativ zu den Rastkugeln (50) des Haltezapfens (20) derart gewählt sind, dass die Rastkugeln (50) mit den Ausfräsungen (44) nach der Axialverstellung und Drehung der Stellhülse (21) in ihre Arretierposition rastend miteinander in Eingriff stehen.

7. Filterhalterung nach einem der Ansprüche 1 bis 6, **dadurch gekennzeichnet, dass** die Andruckscheibe (12) mit dem Haltezapfen (20) feststehend in Verbindung steht.

8. Filterhalterung nach einem der Ansprüche 1 bis 7, **dadurch gekennzeichnet, dass** der Lagerzapfen (11) in seinem zum Wandabschnitt (5) hin liegenden Endbereich einen radial erweiterten Profilsteg (17) mit gleichmäßig am Umfang verteilt angeordneten Halteflächen (18) aufweist und,
dass die Lagerbohrung (34) des Haltezapfens (20) in ihrem zum Wandabschnitt (5) hin liegenden Endbereich ein Lagerprofil (35) aufweist, mit welchem der Haltzapfen (20) in seiner Pressstellung mit den Halteflächen (18) des Profilsteges (17) unverdrehbar in Eingriff steht.

## Claims

1. Filter holder with a sterile container (1) which, in one wall area (5) or in a plurality of wall areas (5, 6), is provided with ventilation openings (7), through which an exchange of air takes place in the closed state between the interior of the sterile container (1) and the environment, wherein the ventilation openings (7) are covered by means of a filter sheet (10) to form a sterile barrier, which effects a sterilization of the air passing through the ventilation openings into the sterile space, wherein the filter sheet (10) is held on the wall area (5, 6) by an air-permeable pressure disc (12),
wherein a mounting pin (11) is provided in the wall area (5, 6) of the ventilation openings (7), which mounting pin (11) protrudes over the wall area (5, 6) and is fixedly connected to the wall area (5, 6),
**characterized in that**
a holding pin (20) with a mounting bore (34) is fitted onto the mounting pin (11), through which holding pin (20) the pressure disc (12) can be pressed in the axial direction (41) against the wall area (5, 6),
the holding pin (20) is connected, in its pressed position, to the mounting pin (11) via a releasable form-fit connection (19, 51),
the form-fit connection (19, 51) can be secured by means of an adjusting sleeve (21) which is mounted displaceably on the holding pin (20).

2. Filter holder according to Claim 1, **characterized in that** the form-fit connection between the mounting pin (11) and the holding pin (20) is composed of a peripheral locking groove (19) of the mounting pin (11) and a plurality of holding balls (51), which are each arranged in a radial through-bore (33) of the holding pin (20), wherein the holding balls (51) are designed to be radially adjustable in the through-bores (33) from a neutral radial position, in which they are not connected to the locking groove (19) of the mounting pin (11), to a blocked position in which they engage in the locking groove (19).

3. Filter holder according to Claim 2, **characterized in that**, for pushing the adjusting sleeve (21) onto the holding pin (20), said adjusting sleeve (21) has a through-bore (40) which, in its end area directed towards the mounting pin (11), has a peripheral, radial expansion (52) which is located in an upper, neutral axial position of the adjusting sleeve (21) on the holding pin (20) in the area of the holding balls (51) and which is dimensioned in terms of its diameter in such a way that the holding balls (51) are movable radially into their neutral position, and,
during an axial adjustment (41) of the adjusting sleeve (21) on the holding pin (20) into its securing position, the through-bore (40) of smaller diameter can be brought into operative connection with the holding balls (51) in such a way that the holding balls (51) can be brought through the through-bore (40) into their blocked position in which they protrude into the mounting bore (34) of the holding pin (20) and engage with the locking groove (19) of the mounting pin (11).

4. Filter holder according to Claims 2 and 3, **characterized in that**, in its outer jacket surface, the holding pin (20) has an L-shaped groove (28), which forms a vertical portion (29) extending parallel to the central longitudinal axis (30) of the holding pin (20), and a horizontal portion (31) extending at right angles thereto, and
a through-bore (46), into which a fixing pin (47) can be fixedly inserted, is provided in the side wall (45) of the adjusting sleeve (21), and
the fixing pin (47), in the mounted state, passes radially inwards through the side wall (45) and engages with the L-shaped groove (28), and
the adjusting sleeve (21) with its fixing pin (47) can be brought from its neutral, upper axial position (41) to its lower securing position along the vertical portion (29) and subsequently, by a relative rotation (59) with respect to the holding pin (20), can be brought with its fixing pin (47) into engagement with the horizontal portion (31) into an axially non-displaceable locking position.

5. Filter holder according to Claim 3 or 4, **characterized in that** the holding pin (20) has, in its upper end area, a continuous and transversely extending through-bore (32), in the radially outer end areas of which locking balls (50) are provided, which are pressed radially outwards by means of an axial compression spring (60), and
the through-bore (40) of the adjusting sleeve (21) has a peripheral fixing groove (42) in its axially upper end area, and
the axial arrangement of the through-bore (32) in the holding pin (29) and the axial arrangement of the fixing groove (42) in the through-bore (40) of the adjusting sleeve (21) are chosen in such a way that the locking balls (50) are in locking engagement with the fixing groove (42) in the upper, neutral axial position of the adjusting sleeve (21).

6. Filter holder according to Claim 5, **characterized in that** the through-bore (40) of the adjusting sleeve (21) has, axially above its fixing groove (42), two mutually diametrically opposite milled recesses (44), and
the circumferential positions and the axial position of the milled recesses (44) of the adjusting sleeve (21) relative to the locking balls (50) of the holding pin (20) are chosen in such a way that the locking balls (50) are in locking engagement with the milled recesses (44) after the axial adjustment and rotation of the adjusting sleeve (21) to its locking position.

7. Filter holder according to one of Claims 1 to 6, **characterized in that** the pressure disc (12) is fixedly connected to the holding pin (20).

8. Filter holder according to one of Claims 1 to 7, **characterized in that** the mounting pin (11) has, in its end area lying towards the wall portion (5), a radially widened profile web (17) with holding surfaces (18) arranged in a uniform distribution on the circumference, and
the mounting bore (34) of the holding pin (20) has, in its end area lying towards the wall portion (5), a bearing profile (35), with which the holding pin (20) engages non-rotatably, in its pressed position, with the holding surfaces (18) of the profile web (17).

## Revendications

1. Support de filtre comprenant un conteneur stérile (1), qui est pourvu, dans une région de paroi (5) ou dans plusieurs régions de paroi (5, 6), d'ouvertures de ventilation (7) à travers lesquelles, dans l'état fermé, un échange d'air a lieu entre l'espace intérieur du conteneur stérile (1) et l'environnement, les ouvertures de ventilation (7) étant recouvertes au moyen d'une feuille de filtre (10) pour former une barrière stérile qui effectue une stérilisation de l'air parvenant dans l'espace stérile à travers les ouvertures de ventilation, la feuille de filtre (10) étant retenue sur la région de paroi (5, 6) par un disque de pressage (12) perméable à l'air, un tourillon de palier (11) faisant saillie au-delà de la région de paroi (5, 6), en liaison fixe avec la région de paroi (5, 6), étant prévu dans la région de paroi (5, 6) des ouvertures de ventilation (7) ;
**caractérisé**
**en ce qu'**un tourillon de retenue (20) est enfiché avec un alésage de palier (34) sur le tourillon de palier (11), le disque de pressage (12) pouvant être pressé par ledit tourillon de retenue contre la région de paroi (5, 6) dans la direction axiale (41),
**en ce que** le tourillon de retenue (20), dans sa position de pressage, est en liaison avec le tourillon de palier (11) par le biais d'une connexion amovible par engagement par correspondance de formes (19, 51),
**en ce que** la connexion par engagement par correspondance de formes (19, 51) peut être sécurisée au moyen d'une douille de réglage (21) supportée de manière déplaçable sur le tourillon de retenue (20).

2. Support de filtre selon la revendication 1, **caractérisé en ce que** la connexion par engagement par correspondance de formes entre le tourillon de palier (11) et le tourillon de retenue (20) est constituée d'une rainure d'encliquetage périphérique (19) du tourillon de palier (11) et de plusieurs billes de retenue (51) disposées à chaque fois dans un alésage traversant radial (33) du tourillon de retenue (20), les billes de retenue (51) dans les alésages traversants (33) étant réalisées de manière déplaçable radialement d'une position neutre radiale non en liaison avec la rainure d'encliquetage (19) du tourillon de palier (11) dans une position de verrouillage s'engageant dans la rainure d'encliquetage (19).

3. Support de filtre selon la revendication 2, **caractérisé en ce que** la douille de réglage (21), pour être poussée sur le tourillon de retenue (20), présente un alésage traversant (40) qui présente, dans sa région d'extrémité orientée vers le tourillon de palier (11), un élargissement radial périphérique (52) qui, dans une position axiale neutre supérieure de la douille de réglage (21) sur le tourillon de retenue (20), se trouve dans la région des billes de retenue (51) et dont le diamètre est dimensionné de telle sorte que les billes de retenue (51) puissent être déplacées radialement dans leur position neutre, et
**en ce que** dans le cas d'un déplacement axial (41) de la douille de réglage (21) sur le tourillon de retenue (20) dans sa position de fixation, l'alésage traversant de plus petit diamètre (40) peut être amené en liaison fonctionnelle avec les billes de retenue (51) de telle sorte que les billes de retenue (51) puissent être amenées à travers l'alésage traversant (40) dans leur position de verrouillage pénétrant dans l'alésage de palier (34) du tourillon de retenue (20) et s'engageant avec la rainure d'encliquetage (19) du tourillon de palier (11).

4. Support de filtre selon les revendications 2 à 3, **caractérisé en ce que** le tourillon de retenue (20) présente dans sa surface d'enveloppe extérieure une rainure en forme de L (28), qui forme une portion verticale (29) s'étendant parallèlement à l'axe médian longitudinal (30) du tourillon de retenue (20) et une portion horizontale (31) s'étendant à angle droit par rapport à celle-ci et **en ce que** dans la paroi latérale (45) de la douille de réglage (21) est prévu un alésage traversant (46) dans lequel peut être insérée fixement une goupille de fixation (47) et
**en ce que** la goupille de fixation (47), dans l'état monté, traverse radialement vers l'intérieur la paroi latérale (45) et est en prise avec la rainure en forme de L (28), et
**en ce que** la douille de réglage (21), avec sa goupille de fixation (47), peut être amenée de sa position axiale neutre supérieure, axialement (41) le long de la portion verticale (29) dans sa position de fixation inférieure et ensuite peut être amenée en prise avec la portion horizontale (31) par une rotation relative (59) par rapport au tourillon de retenue (20) avec sa goupille de fixation (47) dans une position de blocage axialement immobile.

5. Support de filtre selon la revendication 3 ou 4, **caractérisé en ce que** le tourillon de retenue (20), dans sa région d'extrémité supérieure, présente un alésage traversant continu (32) s'étendant transversalement, dans les régions d'extrémité radialement extérieures duquel sont prévues des billes d'encliquetage (50) qui sont pressées radialement vers l'extérieur au moyen d'un ressort de compression axial (60) et
**en ce que** l'alésage traversant (40) de la douille de réglage (21) présente, dans sa région d'extrémité axialement supérieure, une rainure de fixation périphérique (42) et
**en ce que** l'agencement axial de l'alésage traversant (32) dans le tourillon de retenue (29) et l'agencement axial de la rainure de fixation (42) dans l'alésage traversant (40) de la douille de réglage (21) sont choisis de telle sorte que les billes d'encliquetage (50) avec la rainure de fixation (42) soient en prise d'encliquetage les unes avec les autres dans la position axiale neutre supérieure de la douille de réglage (21).

6. Support de filtre selon la revendication 5, **caractérisé en ce que** l'alésage traversant (40) de la douille de réglage (21) présente axialement au-dessus de sa rainure de fixation (42) deux fraisages (44) diamétralement opposés et
**en ce que** les positions périphériques et la position axiale des fraisages (44) de la douille de réglage (21) par rapport aux billes d'encliquetage (50) du tourillon de retenue (20) sont choisies de telle sorte que les billes d'encliquetage (50) soient en prise par encliquetage mutuel avec les fraisages (44) après le déplacement axial et la rotation de la douille de réglage (21) dans sa position de blocage.

7. Support de filtre selon l'une quelconque des revendications 1 à 6, **caractérisé en ce que** le disque de pressage (12) est en liaison fixe avec le tourillon de retenue (20).

8. Support de filtre selon l'une quelconque des revendications 1 à 7, **caractérisé en ce que** le tourillon de palier (11), dans sa région d'extrémité située vers la portion de paroi (5), présente une nervure profilée élargie radialement (17) avec des surfaces de retenue (18) disposées de manière répartie uniformément sur la périphérie et
**en ce que** l'alésage de palier (34) du tourillon de retenue (20) présente, dans sa région d'extrémité située vers la portion de paroi (5), un profil de palier (35) avec lequel le tourillon de retenue (20) est en prise non rotative dans sa position de pressage avec les surfaces de retenue (18) de la nervure profilée (17).
